# EUROPEAN PATENT SPECIFICATION

(11) **EP 1 668 160 B1**
(45) Date of publication and mention of the grant of the patent: **28.04.2010**
(21) Application number: 04784090.5
(22) Date of filing: 13.09.2004
(51) Int. Cl.: C12Q 1/68, G01N 33/543, C12N 15/10

(54) **METHODS, COMPOSITIONS, AND KITS FOR THE CONCENTRATION AND DETECTION OF MICROORGANISMS**
VERFAHREN, ZUSAMMENSETZUNGEN UND KITS ZUR ANREICHERUNG UND ZUM NACHWEIS VON MIKROORGANISMEN
METHODES, COMPOSITIONS, ET TROUSSES POUR CONCENTRATION ET DETECTION DE MICRO-ORGANISMES

(30) Priority: 12.09.2003 US 502368 P
(43) Date of publication of application: 14.06.2006
(73) Proprietor: BioControl Systems, Inc., Bellevue, WA 98005 (US)
(72) Inventor: FELDSINE, Philip, T., Mercer Island, Washington 98040 (US)
(74) Representative: Schiweck, Wolfram
(86) International application number: PCT/US2004/030119
(87) International publication number: WO 2005/028680

(56) References cited:
- EP-A- 0 441 469
- EP-A- 0 626 456
- WO-A-97/09600
- WO-A-97/17611
- WO-A-98/51693
- WO-A-99/42832
- US-A- 5 695 946
- US-B1- 6 265 229
- KULSKI J K ET AL: "USE OF A MULTIPLEX PCR TO DETECT AND IDENTIFY MYCOBACTERIUM AVIUM AND M. INTRACELLULARE IN BLOOD CULTURE FLUIDS OF AIDS PATIENTS" JOURNAL OF CLINICAL MICROBIOLOGY, WASHINGTON, DC, US, vol. 33, no. 3, March 1995 (1995-03), pages 668-674, XP000675402 ISSN: 0095-1137
- WARD, A.C.: "Rapid Analysis of yeast transformants using colony PCR" BIOTECHNIQUES, vol. 13, no. 3, 1992, page 350, XP008104135

## Description

### BACKGROUND OF THE INVENTION

### Field of the Invention

The present invention relates to methods, compositions and kits useful for detecting the presence of a microorganism in a sample. In addition, the present invention relates to methods, compositions and kits useful for preparing a sample for analysis for the presence of a microorganism.

### Description of the Related Art

Nucleic acid amplification methods such as polymerase chain reaction (PCR) have the potential to reduce the time and lower the detection limits of microbiological assays. However, that potential can be achieved only when the sample preparation step yields sufficient amount of purified target nucleic acid to be amplified and detected. Food samples are among the most complex matrices for nucleic acid amplification testing due to the presence of inherent inhibitors such as enzymes, fats and proteins. Environmental samples also represent significant challenges because of solubility issues and potential toxic residues. Many cosmetics and pharmaceutical products contain ingredients, such as anti-microbial agents, which can inhibit microorganism detection. Furthermore, bacterial targets usually occur at very low levels. In processed foods, for example, the bacteria can be stressed, requiring resuscitation to reach detectable levels. As a result, an enrichment step is often necessary for food samples. After enrichment, the sample can then be prepared for the nucleic acid amplification step. A simple, rapid and reliable sample preparation method remains the objective of the diagnostic laboratory.

Many sample preparation methods have been described pertaining to extraction and purification of the sample nucleic acid as a separate step prior to amplification. For example, U.S. Pat. 4,900,677 discloses nucleic acid purification procedure using chaotropes and dialyzed DNA. U.S. Pat. No. 4,923,978 discloses a process for purifying nucleic acid in which a solution of protein and DNA is passed over a hydroxylated support and the protein is bound and the DNA eluted. U.S. Pat. No. 4,935,342 discloses nucleic acid purification by selective binding of DNA to anion exchangers and subsequent elution. U.S. Pat. No. 4,946,952 discloses DNA isolation by precipitation with water-soluble ketones. Other publications (e.g., Holland et al) describe in-house methods and three different commercial DNA extraction methods of extracting bacterial DNA directly from stool specimens for PCR by boiling or addition of lysis buffer. Multiple steps, many times involving centrifugation, are required.

Use of magnetic particles is another technique for the purification of nucleic acids. U.S. Patent 6,534,262 (McKernan, et al) describes a method of selectively isolating a target species of nucleic acid molecules present in a mixture of different size nucleic acid molecules by selectively facilitating the adsorption of a particular species of nucleic acid molecule to the coated surface of magnetic particles. U.S. Pat. 6,433,160 (Collis et al) describes a process for purifying nucleic acid by using a paramagnetic particle in an acidic solution for reversible binding of a nucleic acid molecule without the need for an anionic detergent. Dynal's DNA Direct method describes a process of DNA isolation that relies upon cell lysis and subsequent adsorption of the released DNA to the surface of the Dynabeads during a brief incubation, followed by magnetic separation of the intact DNA/Dynabeads complex, removal of the supernatant and subsequent washing to remove any residual contaminants and potential PCR inhibitors, and then finally resuspension of the complex for direct use in downstream PCR reactions.

Magnetic particles have also been used to capture cells of microorganisms of interest for further detection. U.S. Pat. No. 5491068 and U.S. Pat No. 5,695,946 (Benjamin et al.) describe a method using magnetic beads wherein: 1) the bacteria cells of interest are selectively captured and removed from the sample by the use of an antibody bound to magnetic beads 2) the captured bacteria cells are spread on a medium to form colonies 3) the bacteria colonies are contacted with a colony lift membrane to attach colony material to the membrane; and 4) the presence of colony material from the colonies of bacteria of interest is detected by use of various procedures including nucleic acid amplification. In another method (Dynal publication), the captured cells/particle complex is resuspended and transferred and plated on appropriate culture media, incubated and then counted. In yet another method (Dynal publication), coated beads are added to a cell lysate or other suspension containing the target molecule. After capture, the particles with bound target molecule can be used directly in downstream bioassays, or can be boiled in application buffer and analyzed on SDS-PAGE. Alternately, the target molecule can be eluted off the particles with conventional elution methods.

The methods described above typically require multiple sample preparation steps prior to nucleic acid amplification, including lysis of microorganism cells and/or isolation or purification of nucleic acids. Clearly, there is a need in the art for rapid and reliable methods of preparing and analyzing samples for the presence of microorganisms, including methods of preparing samples for nucleic acid amplification that do not require separate nucleic acid extraction, isolation and purification steps. Furthermore, there is a need in the art for rapid and simple methods of preparing complex samples for subsequent nucleic acid amplification.

### BRIEF SUMMARY OF THE INVENTION

The present invention is directed to methods, compositions and kits, which may be used to prepare samples for nucleic acid amplification and which may be used to detect the presence of a microorganism within a sample.

In one embodiment, the invention provides a method of detecting the presence of a microorganism within a sample. The method includes contacting a sample with an agent that binds to a microorganism or component thereof for a time sufficient to allow the agent to bind the microorganism, concentrating or isolating complexes comprising the agent and the microorganism, performing nucleic acid amplification using one or more primers specific for a polynucleotide of the microorganism in the presence of the complex, and determining the presence of amplified nucleic acids, thereby detecting the presence of the microorganism within the sample. In various embodiments, the methods of the invention further include the step of transferring the microorganisms/concentrating agent complex to a reaction vessel, with or without extraction of the microorganism DNA, prior to performing nucleic acid amplification.

In another embodiment, the invention includes a method of preparing a sample adapted for detecting the presence of a microorganism within the sample by nucleic acid amplification. The method includes contacting a sample with an agent that binds to a microorganism or component thereof for a time sufficient to allow the agent to bind the microorganism and concentrating or isolating complexes comprising the agent and the microorganism.

In yet another related embodiment, the invention includes a method for sample preparation of carrier matrices containing microorganisms of interest for nucleic acid amplification, which includes contacting antibody-coated particles with a carrier matrix to capture microorganisms of interest to create a cell/particle complex, concentrating the cell/particle complex by physical means, and placing the cell/particle complex into a sealed reaction vessel for nucleic acid amplification without extraction of the nucleic acid prior to placement in the reaction vessel.

In certain embodiments of methods of the invention, the agent is associated with a carrier. In specific embodiments, the carrier is a bead, particle, microparticle, insoluble microparticle, magnetic bead, insoluble bead, latex bead, plastic bead, agarose hydrazide bead, agarose bead, sepharose or sephadex.

In related embodiments of methods of the invention, the sample is a food product, environmental sample, water, or beverage.

In one embodiment of the methods of the invention, the microorganism is a bacteria or yeast.

In other embodiments of the invention, the agent is a monoclonal antibody, a polyclonal antibody, or an antibody fragment.

In specific embodiments of methods of the invention, the concentrating or isolation is performed by centrifugation or the use of a magnet.

In related embodiments of methods of the invention, nucleic acid amplification is performed by polymerase chain reaction (PCR), reverse transcription-polymerase chain reaction (RT-PCR) or real-time polymerase chain reaction (real-time PCR).

In one embodiment, the nucleic acid amplification is performed under conditions wherein the microorganism is lysed before or during said amplification.

In another embodiment, the nucleic acid amplification is performed under conditions wherein the microorganism is not lysed before said amplification.

In various embodiments, the nucleic acid amplification is performed under conditions wherein the microorganism is either alive or dead before said amplification is performed.

In related embodiments, nucleic acid amplification is performed in a reaction vessel, which in particular embodiment is a tube, a slide, a plate, a microtitre plate, an array, or a microarray.

In particular embodiments, the microorganisms are bacterial live cells.

In another particular embodiment, the carrier matrix is a food product. In one embodiment, the food product is ground beef.

In yet another embodiment, the invention provides kits for the preparation of a sample for nucleic acid amplification. In one embodiment, a kit includes an agent that specifically binds a microorganism and instructions for use of the kit.

In another embodiment, the invention provides a kit for the preparation of a sample for nucleic acid amplification, which includes an agent that specifically binds a microorganism and one or more primers specific for the microorganism.

In particular embodiments of the kits, the agent is an antibody.

In other embodiments of the kits, the agent is associated with a carrier. In particular embodiment, the carrier is a bead, particle, microparticle, insoluble microparticle, magnetic bead, insoluble bead, latex bead, plastic bead, agarose hydrazide bead, agarose bead, sepharose or sephadex.

In other related embodiments, the kits also include one or more polymerase chain reaction (PCR) reagents. In particular embodiments, the PCR reagents are nucleotides, buffers or polymerases.

In another embodiment, the invention includes a method of detecting the presence of a microorganism within a sample, comprising diluting a sample in a liquid medium that supports growth of a microorganism; incubating the sample in the liquid medium under conditions and for a time sufficient to allow growth of the microorganism; contacting the sample in the liquid medium following step or an aliquot thereof, with an agent that bind to the microorganism or component thereof for a time sufficient to allow the agent to bind the microorganism; isolating complexes comprising the agent and the microorganism; nucleic acid amplification using one or more primers specific for a polynucleotide of the microorganism in the presence of the complex; and determining the presence of amplified nucleic acids and thereby detecting the presence of the microorganism in the sample. In various embodiments of this method, the liquid medium comprises mEHEC medium, the binding agent is an antibody specific for *E. coli* 0157, and/or the microorganism is *E. coli* 0157.

In certain embodiments of the invention, the binding agent is associated with magnetic beads, and isolation is performed using a magnet.

In specific embodiments of detection methods of the invention, two or more aliquots of sample in liquid media are each incubated with a different binding agent. In a certain embodiment, the different binding agents are specific for different microorganisms.

In other specific embodiments, nucleic acid amplification is performed using two or more sets of primers capable of amplifying a polynucleotide of a microoganism. Each set of primers may be capable of amplifying polynucleotides of different microorganisms, or each set of primers may be capable of amplifying polynucleotides of the same microorganism.

### DETAILED DESCRIPTION OF THE INVENTION

The present invention provides novel methods, compositions and kits that are useful in the preparation of samples for nucleic acid amplification. The methods, compositions and kits of the invention may be used in a variety of ways and are particularly valuable for use in the detection of the presence of one or more microorganisms in a sample by nucleic acid amplification. In certain embodiments, the methods of the invention provide for the concentration or isolation of microorganisms from a sample, such as, e.g., a matrix, as described herein, thereby facilitating detection of the microorganisms via nucleic acid amplification. The methods and kits of the present invention provide significant advantages over current technologies, including, in certain embodiments, providing rapid and reliable means of detecting a microorganism in a sample, without the need for lysing cells or purifying nucleic acids from cells prior to nucleic acid amplification.

The skilled artisan would appreciate that the present invention may be used to detect a wide variety of microorganisms from any of a large number of samples. Accordingly, the invention is not limited to the specific components, such as microorganisms, samples, and binding agents, described herein.

### Sample Preparation and Amplification Methods

Methods of the present invention may be used to prepare a sample for a nucleic acid amplification procedure. Methods of the present invention may also be used to detect the presence of a microorganism within a sample. Accordingly, methods of the present invention have variety of related uses, including detecting or diagnosing an infection or disease associated with a particular microorganism, determining the amount of microorganism present within a sample, particularly when using quantitative nucleic acid amplification procedures, such as quantitative or real-time polymerase chain reaction, and determining whether a threshold level of a microorganism is present within a sample.

A threshold level depends, in large part, upon the sample being tested, the type of microorganism suspected of being present in the sample, and any industry or government-imposed standards related to maximum microorganism concentration. The determination of an appropriate threshold level for a particular sample to be tested may readily be determined by the skilled artisan based upon these and any other criteria established for a suitable application. In one embodiment, a threshold level is the maximum level considered acceptable by government or industry regulatory standards. In certain embodiments, the threshold level is 1 x 10² organisms/ml, 5 x 10² organisms/ml, 1 x 10³ organisms/ml, 5 x 10³ organisms/ml, 1 x 10⁴ organisms/ml, 5 x 10⁴ organisms/ml, 1 x 10⁵ organisms/ml, or 5 x 10⁵ organisms/ml, or any integer value falling between.

Detection of a microorganism may also be determined based upon the presence of an increased amount of a microorganism in sample as compared to a control sample. In certain embodiments, the presence if a microorganism is detected if the sample contains at least two, at least three, at least four, at least five or at least ten-fold more of a microorganism than a control sample. Depending upon the nature of detection being performed, in certain embodiment, a control sample may be a sample known to not contain the microorganism being detected (e.g., a buffer solution), or it may be a sample containing a pre-determined or acceptable amount of a microorganism. In addition, the detection or a diagnosis of a disease may be associated with an increased or decreased level of one or more microorganisms in a sample as compared to a control sample from a patient that does not have the disease. Accordingly, disease may be detected or diagnosed based upon any relevant increase or decrease in microorganism number. For example, a disease may be detected or diagnosed if the sample contains at least two, at least three, at least four, at least five or at least ten-fold more of a microorganism than a control sample that is known to be absent the disease.

In one embodiment of the invention directed to the detection of a microorganism within a sample, the sample is contacted or incubated with an agent that binds to one or more microorganism or components thereof, generally for a time sufficient to permit binding of the agent to the microorganism. Such times may be readily determined by the skilled artisan, depending upon the particular type of binding agent being used. In certain embodiments, the agent will be conjugated or associated with a carrier, as described below. In other embodiment, the agent will not be associated with a carrier.

Complexes of binding agent and microorganism may then be isolated, purified, or concentrated via the binding agent or associated carrier. Typically, the concentration or purification employs a physical means, such as a centrifuge or magnet. For example, if the carrier is a magnetic bead, then the complexes may be isolated or concentrated through the use of standard procedures employing a magnet. In another embodiment, the carrier and associated complexes are isolated or concentrated via centrifugation. In another example, wherein the binding agent is an antibody, purification or concentration is accomplished through the use of a secondary antibody conjugated to a bead or through the use of an agent such as protein A sepharose beads or protein G sepharose beads, which bind antibodies. A variety of purification and concentration methods are known in the art, and all are suitable for practice according to the present invention. In specific embodiments, antibody-coated insoluble beads or antibody-coated magnetic beads are used for purification or concentration. The purified or concentrated complex may be washed, e.g., to remove contaminants that might interfere with subsequent nucleic acid amplification or change buffers.

In one embodiment, a binding agent (e.g., an antibody) is attached to magnetic particles or magnetic beads, e.g., Dynabeads® M-270 (Dynal; Lake Success, NY), and these magnetic beads may be isolated using a magnet or device as described in U.S. Patent No. 6,468,810. In one particular embodiment, the device used is a PickPen™ (Bio-Nobile; Turku, Finland), available from BioControl Systems, Inc. (Bellevue, WA).

The purified or concentrated complex may then used as a template or substrate for nucleic acid amplification. In one embodiment, the complex is transferred to a reaction vessel suitable for the particular nucleic acid amplification method being employed. However, in another embodiment, the nucleic acid amplification is performed in the same vessel used for purification or concentration. Suitable vessels, depending upon the method employed, include, e.g., test tubes, microfuge tubes, microtitre plates, slides, plates, arrays, and microarrays. Multiple samples may be tested or a single sample tested for multiple microorganisms through the use of arrays, such as microarrays. Accordingly, the invention includes high throughput methods employing the methods described herein, including, e.g., high throughput PCR methods.

Any of a variety of different nucleic acid amplification procedures known in the art may be used to detect or determine the presence of a microorganism within a sample. Examples of nucleic acid amplification methods include polymerase chain reaction (PCR) methods, including, e.g., reverse transcription-PCR and real-time PCR. In addition, the invention may be practiced using other amplification methods such as, e.g., nuclear run-offs, primer extension, RNase protection, and rolling circle amplification methods, all of which are widely known and available in the art and are described, e.g., in Sambrook, et al. Molecular Cloning: A Laboratory Manual (2nd Edition, 1989) and Maniatis et al. Molecular Cloning: A Laboratory Manual (1982).

In one embodiment, PCR is performed using the Rotorgene™ by Corbett Research of Sydney, Australia, which performs affordable and accurate real-time PCR.

Nucleic acid amplification is performed in the presence of one or more oligonucleotides or primers capable of specifically binding to a polynucleotide of a microorganism. Such binding may be under high or moderately stringent conditions. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-60°C, 5 X SSC, overnight; followed by washing twice at 65°C for 20 minutes with each of 2X, 0.5X and 0.2X SSC containing 0.1 % SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, *e*.*g*., to 60-65°C or 65-70°C. PCR-based amplification methods may be performed using two primers capable of amplifying a region of a microorganism polynucleotide. In certain embodiments, the polynucleotide is genomic DNA. In another embodiment, reverse-transcription PCR may be employed in the amplification of cDNA generated from microorganism RNA. In certain embodiments, primers comprise at least 12, at least 15, at least 18, at least 21, or at least 24 nucleotides corresponding to or complementary to a region of a polynucleotide within a microorganism.

In related embodiments, the invention provides a method of detecting the presence of a microorganism within a cell, which includes isolating or concentrating a cell as described above in general for microorganisms and then performing nucleic acid amplification using primers specific for or capable of amplifying a region of microorganism polynucleotide sequence.

PCR cycling methods are well known and described in the art. In one embodiment of the invention, nucleic acid amplification methods are employed that are capable of lysing cells present within the sample. Such cells may be the microorganism being detected or may contain the microorganism being detected. In one embodiment, cell lysis involves an initial heating step prior to thermocycling sufficient to lyse the cells. However, in other embodiment, thermocycling conditions are selected that lyse cells within one or more cycles. Such methods are known in the art and have been previously described, e.g., in methods related to PCR-based analysis of bacteria, e.g., PCR minipreps.

The present invention also includes methods of preparing a sample for subsequent nucleic acid amplification procedures.

In a specific embodiment, the present invention provides a sample preparation method suitable for use with nucleic acid amplification detection that uses insoluble micro-particles to capture cells and then uses the cell/particle complex directly for nucleic acid amplification. In certain embodiments, this method does not require a separate washing, cell lysis, nucleic acid extraction or purifying step, and the cell/particle complex does not need to be transferred to another cultural medium for growth.

In another specific embodiment, the invention provides a method of capturing microorganisms of interest and removing them from a carrier matrix by the use of antibody-coated insoluble beads. In one preferred embodiment, the beads are magnetic particles, concentrating the cell/particle complex by physical means, removing the supernatant and resuspending the cell/particle complex, transferring the cell/particle complex directly into a sealed reaction vessel such as a PCR tube, and conducting nucleic acid amplification detection assay under conditions such that the cells are lysed and DNA released in the reaction vessel during the first few cycles of amplification without opening the sealed reaction vessel.

In certain embodiments of methods of the invention, sample preparation includes a dilution and enrichment step. The dilution and enrichment step generally includes suspending a sample in a solution, typically a growth supportive media such as, e.g., mEHEC, LB, Terrific Broth, YT, EHEC8, which selectively cultivates *E. coli* 0157 and other closely related lactose-fermenting microorganisms (Biocontrol; Bellevue, WA), SOB, or SOB, or a dilution thereof, and allowing the sample in the solution to incubate at an appropriate temperature to facilitate growth of a microorganism within the sample, such as e.g., 20 °C, 25 °C, 30°C, 37° C, or 42 °C. In one specific embodiment, enrichment is carried out by placing a sample in diluted EHEC8 media and incubating it for 6 hours at 42 °C. In one embodiment, the dilution and enrichment step permits amplification of the amount of a microorganism in a sample and, thereby, facilitates detection of the microorganism. In another embodiment, the dilution and enrichment step is advantageous for solid or matrix samples, where dilution facilitates the release and subsequent detection of a microorganism. Accordingly, in certain embodiments, sample preparation prior to amplification comprises the steps of: diluting a sample and/or enriching a microorganism within a sample.

In one specific embodiment of a method of detecting a microorganism of the present invention, a sample is diluted and a microorganism is enriched by placing the sample into a liquid comprising a media, such as, e.g., mEHEC media and incubating the diluted sample for approximately 2-24 h, 4-8 h, or 6 h at a temperature that supports microorganism growth, such as, e.g., 37 °C or 42°C. The diluted sample, or a portion thereof, is incubated with magnetic beads having an attached antibody specific for a microorganism of interest, such as, e.g., anti-E. coli 0157 antibody (available from Fitzgerald; Concord, MA). Following incubation, the beads, and any bound microorganisms, are isolated from solution using a magnet or magnet device, such as the PickPen. The beads may optionally be washed to remove unbound cells and other materials. The beads, or microorganisms attached to the beads, are then placed into reaction tubes for subsequent PCR analysis using primers specific for a microorganism of interest.

It is understood that the methods of the invention may be used to detect a single microorganism or multiple different microorganisms. Accordingly, aliquots of sample (before or after dilution and enrichment) may be incubated with a variety of different binding agents specific for one or more different microorganisms and then processed via PCR using primers specific for one or more different organisms to determine the presence, absence or amount of one or more microorganisms. Therefore, in one embodiment, aliquots of sample are incubated with a binding agent specific for a microorganism and the presence of bound microorganism is subsequently detected using various sets of primers specific for the microorganism. In another related embodiment, aliquots of a sample are incubated with a panel of binding agents specific for different microorganisms and the presence of bound microorganism is detected using sets of primers specific for each of the bound microorganisms. In another related embodiment, the binding agent may bind a broad class of microorganisms, such as, e.g., gram positive or gram negative bacteria, and the presence of specific microorganisms is determined by performing PCR using primers specific for such microorganisms.

### Samples

The present invention may be used to detect the presence of one or more microorganisms in a variety of samples. In a related embodiment, the invention may be use to prepare any of a wide variety of samples for analysis for the presence of a microorganism. In certain embodiments, the invention is useful for preparing or detecting the presence of a microorganism in a sample that is a matrix. A matrix may also be referred to as a carrier matrix, which indicates that a microorganism is present within the matrix. According to the invention, a matrix sample is a sample comprising components in addition to the microorganism. A complex matrix sample comprises multiple components in addition to the microorganisms. In certain embodiments, a matrix sample is a solid matrix sample and comprises a solid component. In specific embodiments, the additional components of a matrix sample may interfere with or inhibit amplification of nucleic acids of the microorganism. In another embodiment, an additional component may present a physical barrier to obtaining or isolating nucleic acids. The additional components may also reduce the possibility of successful nucleic acid amplification of microorganisms present within the matrix sample due to their diluting effect, which effectively reduces the concentration of microorganism present within the sample. Examples of matrix samples include, but are not limited to, food, beverages, pharmaceutical compositions, and cosmetics.

In certain embodiments, the invention contemplates the use of liquid and/or solid samples. In certain embodiments, solid samples are suspended or dissolved in a liquid. In another embodiment, a solid sample may be soaked in a liquid to release microorganisms from the sample into the liquid, and the liquid may then be tested or prepared according to the methods of the invention.

In certain embodiments, the invention may be used to detect the presence of one or more microorganisms in any beverage or food. Examples of beverages include, but are not limited to, milk products, *e*.*g*., milk and cream; alcoholic beverages, *e*.*g*. beer and wine; juices, and water. Water includes, but is not limited to, drinking or potable water. Examples of food products include, but are not limited to, baby foods, packaged foods, frozen foods, eggs, dairy products, including, e.g., yogurts and cheeses, and meats and ground meats, including, e.g., poultry, pork, beef, and lamb.

In another embodiment, the sample is a tissue, e.g., human tissue, or bodily fluid such as blood, urine, spinal fluid, or other similar fluids. In a related embodiment, the invention may be used in the detection or preparation of a sample for detecting the presence of a microorganism in blood products and pharmaceutical preparations. For example, the invention may be used to detect bacteria in preparations comprising whole blood, platelets, red blood cells, and/or leukocytes, including concentrates suitable for transfusion.

In another embodiment, the invention may be used to detect the presence of a microorganism in an environmental sample. An environmental sample may be from any source. Samples may be found outdoors or indoors. Examples of environmental samples include, but are not limited to, dirt, dust, plants, and water samples. Water from any source may be tested according to the invention, including, *e*.*g*., water from swimming pools, heating and cooling systems, and natural or outdoor waters, *e*.*g*., lakes and rivers. In one embodiment, which may be related to the detection of a biowarfare agent, the sample may be from an unknown source and may be a powder or liquid or air particulates, for example.

In certain embodiments, samples include pharmaceutical preparations, cosmetics, or personal hygiene products.

### Microorganisms

The methods, compositions, and kits of the present invention may be used to prepare samples containing or suspected of containing any of a large number of microorganisms. Accordingly, the present invention may be used to isolate and/or detect the presence of any microorganism of interest within a sample. In certain embodiments, the present invention is used to prepare samples for or detect the presence of a pathogenic microorganism in a sample.

Examples of microorganisms include, but are not limited to, bacteria (including both gram-positive and gram-negative bacteria), bacterial spores, yeast, other fungi, viruses, and bacteriophage. In specific embodiments of the invention, microorganisms are cells, while in other embodiments, microorganism may be present within cells. Accordingly, in one particular embodiment of the invention, an agent that binds to a cell comprising a microorganism is used in combination with primers that amplify nucleic acids of the microorganism present within the cell. In certain embodiments, microorganisms are viable cells, while in other embodiments, microorganisms are dead cells. Viable cells are cells that have intact cell membranes and are actually or potentially metabolically active. As used herein, spores and cysts are examples of viable cells. In other embodiments, microorganisms are non-viable cells.

In certain embodiments, the invention may be used to detect any infectious agent. As used herein, an "infectious agent" is any living organism capable of infecting a host. Infectious agents include, for example, bacteria, viruses, fungi, and protozoa. The infectious agent may be a biowarfare agent. Biowarfare agents include viruses, bacteria, fungi and toxins having the capability to produce death or disease in humans, animals or plants. Examples of infectious agents include, but are not limited to, Actinobacillus spp., Actinomyces spp., Adenovirus (types 1, 2, 3, 4, 5 et 7), Adenovirus (types 40 and 41), Aerococcus spp., Aeromonas hydrophila, Ancylostoma duodenale, Angiostrongylus cantonensis, Ascaris lumbricoides, Ascaris spp., Aspergillus spp., Bacillus anthracis, Bacillus cereus, Bacteroides spp., Balantidium coli, Bartonella bacilliformis, Blastomyces dermatitidis, Bluetongue virus, Bordetella bronchiseptica, Bordetella pertussis, Borrelia burgdorferi, Branhamella catarrhalis, Brucella spp. (B. abortus, B. canis, B. melitensis, B. suis), Brugia spp., Burkholderia, (Pseudomonas) mallei, Burkholderia (Pseudomonas) pseudomallei, California serogroup, Campylobacter fetus subsp. Fetus, Campylobacter jejuni, C. coli, C. fetus subsp. Jejuni, Candida albicans, Capnocytophaga spp., Chlamydia psittaci, Chlamydia trachomatis, Citrobacter spp., Clonorchis sinensis, Clostridium botulinum, Clostridium difficile, Clostridium perfringens, Clostridium tetani, Clostridium spp. (with the exception of those species listed above), Coccidioides immitis, Colorado tick fever virus, Corynebacterium diphtheriae, Coxiella burnetii, Coxsackievirus, Creutzfeldt-Jakob agent, Kuru agent, Crimean-Congo hemorrhagic fever virus, Cryptococcus neoformans, Cryptosporidium parvum, Cytomegalovirus, Dengue virus (1, 2, 3, 4), Diphtheroids, Eastern (Western) equine encephalitis virus, Ebola virus, Echinococcus granulosus, Echinococcus multilocularis, Echovirus, Edwardsiella tarda, Entamoeba histolytica, Enterobacter spp., Enterovirus 70, Epidermophyton floccosum,, Microsporum spp. Trichophyton spp., Epstein-Barr virus, Escherichia coli, enterohemorrhagic, Escherichia coli, enteroinvasive, Escherichia coli, enteropathogenic, Escherichia coli, enterotoxigenic, E.coli spp., E.coli 0157, E.coli 055:H7, E.coli 0111 and E.coli 0126, Fasciola hepatica, Francisella tularensis, Fusobacterium spp., Gemella haemolysans, Giardia lamblia, Haemophilus ducreyi, Haemophilus influenzae (group b), Hantavirus, Hepatitis A virus, Hepatitis B virus, Hepatitis C virus, Hepatitis D virus, Hepatitis E virus, Herpes simplex virus, Herpesvirus simiae, Histoplasma capsulatum, Human coronavirus, Human immunodeficiency virus, Human papillomavirus, Human rotavirus, Human T-lymphotrophic virus, Influenza virus, Junin virus /Machupo virus, Klebsiella spp., Kyasanur Forest disease virus, Lactobacillus spp., Legionella pneumophila, Leishmania spp., Leptospira interrogans, Listeria monocytogenes, Listeria spp, oriomeningitis virus, Marburg virus, Measles virus, Micrococcus spp., Moraxella spp., Mycobacterium spp. (other than M. bovis, M. tuberculosis, M. avium" M. leprae), Mycobacterium tuberculosis, M. bovis, Mycoplasma hominis, M. orale, M. salivarium, M. fermentans, Mycoplasma pneumoniae, Naegleria fowleri, Necator americanus, Neisseria gonorrhoeae, Neisseria meningitides, Neisseria spp. (other than N. gonorrhoeae and N. meningitidis), Nocardia spp., Norwalk virus, Omsk hemorrhagic fever virus, Onchocerca volvulus, Opisthorchis spp., Parvovirus B19, Pasteurella spp., Peptococcus spp., Peptostreptococcus spp., Plesiomonas shigelloides, Powassan encephalitis virus, Proteus spp., Pseudomonas spp. (other than P. mallei, P. pseudomallei), Rabies virus, Respiratory syncytial virus, Rhinovirus, Rickettsia akari, Rickettsia prowazekii, R. Canada, Rickettsia rickettsii, Ross river virus / O'Nyong-Nyong virus, Rubella virus, Salmonella choleraesuis, Salmonella paratyphi, Salmonella typhi, Salmonella spp. (with the exception of those species listed above), Schistosoma spp., Scrapie agent, Serratia spp., Shigella spp., Sindbis virus, Sporothrix schenckii, St. Louis encephalitis virus, Murray Valley encephalitis virus, Staphylococcus aureus, Streptobacillus moniliformis, Streptococcus agalactiae, Streptococcus faecalis, Streptococcus pneumoniae, Streptococcus pyogenes, Streptococcus salivarius, Taenia saginata, Taenia solium, Toxocara canis, T. cati, Toxoplasma gondii, Treponema pallidum, Trichinella spp., Trichomonas vaginalis, Trichuris trichiura, Trypanosoma brucei, Ureaplasma urealyticum, Vaccinia virus, Varicella-zoster virus, Venezuelan equine encephalitis, Vesicular stomatitis virus, Vibrio cholerae, serovar 01, Vibrio parahaemolyticus, West Nile virus, Wuchereria bancrofti, Yellow fever virus, Yersinia enterocolitica, Yersinia pseudotuberculosis, and Yersinia pestis.

The methods, kits, and compositions may be used to prepare samples for the detection of microorganisms associated with bacterial contamination of blood products, such as platelets. Examples of such microorganisms include, but are not limited to, staphylococcus aureus, Klebsiella pneumoniae, Serratia marcescens, and Staphylococcus epidermidis. Other isolated organisms include Salmonella sp., Escherichia coli, Pseudomonas aeruginosa, and Bacillus cereus.

In certain embodiments, microorganisms are biowarfare agents, including bacteria and fungi, for example. Examples of bacteria and spores that may be detected according to the present invention include, but are not limited to, Bacillus anthracis, Bacillus cereus, Clostridium botulinum, Yersinia pestis, Yersinia enterocolitica, Francisella tularensis, Brucella species, Clostridium perfringens, Burkholderia mallei, Burkholderia pseudomallei, Staphylococcus species, Tuberculosis species, Escherichia coli, Group A Streptococcus, Group B streptococcus, Streptococcus pneumoniae, Helicobacter pylori, Francisella tularensis, Salmonella enteritidis, Mycoplasma hominis, Mycoplasma orale, Mycoplasma salivarium, Mycoplasma fermentans, Mycoplasma pneumoniae, Mycobacterium bovis, Mycobacterium tuberculosis, Mycobacterium avium, Mycobacterium leprae, Rickettsia rickettsii, Rickettsia akari, Rickettsia prowazekii, Rickettsia canada, and Coxiella burnetti.

Examples of yeast and other fungi microorganisms include, but are not limited to, Aspergillus species (e.g. Aspergillus niger), Mucor pusillus, Rhizopus nigricans, Candida species (e.g. Candida albicans, Candida dubliniensis, C. parapsilosis, C. tropicalis, and C. pseudotropicalis), Torulopsis glabrata, Blastomyces dermatitidis, Coccidioides immitis, Histoplasma capsulatum, Cryptococcus neoformans, and Sporothrix schenckii.

### Binding Agents

Any of a variety of agents that bind a microorganism may be used according to the present invention, including, for example, polypeptides, sugars, and nucleic acids. In preferred embodiments of the invention, agents specifically bind one or more microorganisms.

In certain embodiments, the binding agent is an antibody specific for a microorganism. In other embodiments, the antibody is a monoclonal antibody, a polyclonal antibody, or a fragment thereof. Within the context of the present invention, antibodies are understood to include monoclonal antibodies, polyclonal antibodies, anti-idiotypic antibodies, humanized antibodies, Primatized™ antibodies, and antibody fragments (*e*.*g*., Fab, and F(ab')₂, Fᵥ variable regions, or complementarity determining regions), for example. Antibodies are generally accepted as specific if they bind with a K_{d} of greater than or equal to 10⁻⁷M, preferably greater than of equal to 10⁻⁸M. The affinity of a monoclonal antibody or binding partner can be readily determined by one of ordinary skill in the art (see Scatchard, Ann. N.Y. Acad. Sci. 51:660-672, 1949). Once suitable antibodies have been obtained, they may be isolated or purified by many techniques well known to those of ordinary skill in the art.

Antibodies may be produced by any means available in the art. For example, antibodies against microorganisms or cell surface molecules may be raised by immunization of mice, rats, rabbits or other animals with microorganisms, peptides, polypeptides, or cells (or membrane preparations thereof). Various immunization protocols may be found in, for example, Harlow and Lane Antibodies: A Laboratory Manual, Cold Spring Harbor Laboratory, 1988 and Coligan et al. Current Protocols in Immunology, Greene Publishing, 1995. Following immunization, spleen or lymph nodes are collected for generating hybridomas or serum is collected for polyclonal antibodies. Hybridomas may be generated by any method available in the art (see, U.S. Patent Nos. RE 32,011, 4,902,614, 4,543,439, and 4,411,993; Harlow and Lane, *supra*; and Coligan *et al., supra*; for protocols). Antibody-secreting hybridomas are grown, and the antibodies are tested for binding to the immunizing microorganisms, peptides, polypeptides, or cells by ELISA, section staining, flow cytometry, confocal microscopy and the like. In one embodiment, the binding agent or a primer used for nucleic acid amplification is labeled or comprises a label that is detectable either directly or indirectly. In certain embodiments, a label may be a fluorescent compound, which can respond to applied electromagnetic energy, such as ultraviolet or visible light, to provide an emitted signal that can be detected visually or detected instrumentally. Labels include those used in fluorescence resonance energy transfer (FRET)-based detection methods, such as, *e*.*g*., fluorescein and rhodamine.

In certain embodiment, the agent binds to one or more bacteria. In one embodiment, the binding agent specifically binds to the cell surface of bacteria or to a component thereof. The binding agent may be specific for one or more different types or stains of bacteria. In certain embodiments, the binding agent binds to gram-positive or gram-negative bacteria or both. In certain embodiments, the binding agent or antibody recognizes all or a large number of different bacteria. In certain embodiments, the binding agent is directed against a component of the bacterial surface. In one particular embodiment, the binding agent is an antibody specific for E. coli 0157. In another embodiment, the binding agent is an antibody specific for Listeria, including Listeria spp and Listeria monocytogenes. In another embodiment, the binding agent is an antibody specific for Salmonella spp. In still another embodiment, the binding agent is an antibody specific for Campylobacter spp. Such antibodies are commercially available from producers and distributors, including, e.g., Fitzgerald Industries; Concord, MA and East Coast Biologics; North Berwick, ME. In one embodiment of the invention, two or more agents that bind to microorganisms are used. These agents may bind to the same or different microorganisms. For example, several agents that each specifically bind to different pathogenic bacteria may be used in combination according to the invention, in order to determine the presence of any or all of such bacteria in a sample.

In a preferred embodiment of the invention, the agent that binds a microorganism (or cell comprising a microorganism) is coupled or conjugated to a carrier, which may be used to facilitate the concentration, isolation, or purification of the microorganism or cell. A wide variety of suitable carriers are known and available in the art. The skilled artisan would be able to readily determine the appropriateness of a particular carrier for use with a particular binding agent. Examples of carriers that may be used according to the present invention include, but are not limited to, beads, particles, microparticles, insoluble microparticles, magnetic beads, insoluble beads, latex beads, plastic beads, agarose hydrazide beads, agarose beads, sepharose and sephadex. In one embodiment of the invention, the carrier is insoluble, although in another embodiment, the carrier is soluble. Such reagents are widely commercially available.

In certain embodiments, carriers are physical supports, such as beads or microparticles and facilitate concentration or isolation of bound microorganisms or cells, e.g, via centrifugation. In another embodiment, carriers are metal or magnetic beads that facilitate concentration or isolation of bound microorganisms or cells, e.g., via the use of a magnet.

In one particular embodiment, a carrier and binding agent combination includes a bead coated with an antibody, such as a monoclonal antibody, specific for a microorganism or cell.

### Compositions and Kits

The present invention further provides compositions and kits that may be used according to one or more methods of the invention. For example, in certain embodiments, kits of the present invention are adapted for preparing a sample for nucleic acid amplification, and in other embodiments, kits of the present invention are adapted for detecting the presence of a microorganism in a sample. Kits of the invention may include instructions for their use. As described below, a variety of different components may be present within a kit of the present invention. The invention includes kits containing each and every combination of components, including the specific combinations and embodiments described below.

In certain embodiments, a kit for the preparation of a sample for nucleic acid amplification includes one or more agents that specifically bind a microorganism. In certain embodiment, this agent is conjugated to a carrier, which typically facilitates isolation or concentration of bound microorganisms. In a specific embodiment, the agent is an antibody, such as a monoclonal or polyclonal antibody or fragment thereof. Kits may be adapted for the detection of one or more specific microorganisms by including one or more agents that specifically bind one or more or the selected microorganisms. Kits may also be adapted for the detection of multiple microorganisms by including an agent that binds to multiple microorganisms.

In other embodiments, kits for the detection of a microorganism include one or more primers suitable for nucleic acid amplification of a microorganism polynucleotide. Methods of selecting and preparing primers suitable for nucleic acid amplification procedure, such as polymerase chain reaction, are widely known and available in the art. In one embodiment, the primer(s) are specific for polynucleotides of the same microorganism(s) or a selected set of one or more microorganisms that are specifically bound by the agent included in the kit. In alternative embodiments, such as where the microorganism to be detected may be present within a cell present in the sample being tested, the primers may be specific for one or more microorganisms present or suspected of being present in the cell, and the agent specifically bind the cell.

Kits of the present invention may also optionally include reagents for nucleic acid amplification, such as, for example, one or more nucleotides or analogs thereof, buffers, and polymerases, such as a thermostable polymerase, e.g., a taq polymerase.

Compositions of the invention include any of the reagents employed to practice the methods of the invention, including, e.g. binding agent conjugated to carriers.

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, *e*.*g*., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

All of the above U.S. patents, U.S. patent application publications, U.S. patent applications, foreign patents, foreign patent applications and non-patent publications referred to in this specification and/or listed in the Application Data Sheet, and references and patents cited therein, are incorporated herein by reference, in their entirety.

### EXAMPLE 1

### DETECTION OF BACTERIA IN A SAMPLE

The presence or absence of bacteria in a sample is determined according to the following procedure employing the steps of sample preparation and amplification and detection.

A sample is prepared for subsequent amplification and detection by suspending the sample in Sample Dilution and Enrichment Media, incubating the sample with Dynal M-270 magnetic beads having attached antibodies specific for a bacteria being detected, and isolating the magnetic beads and any associated bacteria bound to the attached antibodies, according to the following steps.

Sample Dilution and Enrichment Media is prepared by suspending 7.1 g mEHEC media in 225 ml of pre-warmed (42°C) sterile water. Approximately 25 g of sample is added to the sample dilution and enrichment media and incubated at 42° C for approximately six hours, resulting in Enriched Sample. Sample Reagent is prepared by diluting the magnetic beads with attached antibodies at a ratio of between 1:1 and 1:50 in buffer at a pH of 6.5 to 9.0 with between 0.0001 and 1% protein.

20 µl of Sample Reagent is placed into each well of a 96-well plate (Sample Block), and 1 ml of the Enriched Sample is added to each well. The Sample Block is covered with adhesive film and vortex mixed at 900 rpm for 5 minutes.

25 µl of Resuspension Buffer consisting of any non-phosphate buffer below 50 mm concentration is placed into each well of a second 96-well plate (Suspension Plate). A PickPen™ is inserted into the first row of the Sample Block with the magnets extended and stirred with a corkscrew up and down motion for 30 seconds, thereby facilitating binding of the magnetic beads to the PickPen™ magnets. The PickPen™ is then inserted into the first row of the Suspension plate, and the magnets are retracted to release the beads into the Resuspension Buffer. This procedure is repeated for all rows of the Sample Block.

Taq Buffer is prepared by diluting Taq® enzyme to the appropriate unit concentration in Taq Dilution Buffer consisting of Tris buffer, pH 8.7; 10-100 mm sodium chloride; 0.2 - 5 M betaine; and 2 - 40% glycerol.

Amplification of polynucleotides in bacteria bound to the magnetic beads is performed by adding 5 µl of Taq Buffer to each PCR tube cooled to 2-8° C. 20 µl of liquid from each well of the Suspension Plate is transferred into a separate prepared PCR tube. The PCR tubes are placed into a Rotorgene, and amplification is performed. The amplification mixture contains forward and reverse primers to genes specific for the bacteria being detected; a fluorescent probe specific for the same bacterial gene target; forward and reverse primers, and a fluorescent probe for an amplification control; between 10 and 10,000 copies of the amplification control gene; and necessary salts for the amplification reaction; all in a lyophilized mixture. As the amplification proceeds, the presence of the bacterial specific gene is determined by the increase in fluorescence at the wavelength specific for the fluorescent probe that binds to the bacterial gene target. In the absence of bacterial gene target, there is no signal at this wavelength and the signal at the wavelength for the fluorescent probe that binds to the amplification control gene indicates that a valid reaction has occurred.

From the foregoing it will be appreciated that, although specific embodiments of the invention have been described herein for purposes of illustration, various modifications may be made without deviating from the spirit and scope of the invention.

## Claims

1. A method of detecting the presence of a microorganism within a sample, wherein the microorganism is a bacterium, the method comprising:
(a) contacting a sample with a carrier having attached antibodies that bind to the bacterium or component thereof for a time sufficient to allow the antibodies to bind the bacterium;
(b) isolating the carrier and any associated bacteria bound to the attached antibodies;
(c) transferring the complex of step (b) to a reaction vessel and directly performing nucleic acid amplification using: forward and reverse primers specific for a gene specific for the bacterium being detected wherein the amplified nucleic acid is genomic DNA, wherein said bacterium is bound to the carrier, and wherein the bacterium is lysed under thermocycling conditions, such that lysis is achieved within one or more cycles; and wherein said amplification is performed by polymerase chain reaction (PCR), wherein step (c) directly succeeds step (b); and
(d) determining the presence of amplified nucleic acids and thereby detecting the presence of the bacterium within the sample.

2. The method of claim 1, wherein the carrier is selected from the group consisting of: beads, particles, microparticles, insoluble microparticles, magnetic beads, insoluble beads, latex beads, plastic beads, agarose hydrazide beads, agarose beads, sepharose and sephadex.

3. The method of claim 1 or 2, wherein the sample is selected from the group consisting of: food products, environmental samples, water, and beverages.

4. The method of any one of claims 1 to 3, wherein the antibody is selected from the group consisting of: monoclonal antibodies, polyclonal antibodies, and antibody fragments.

5. The method of any one of claims 1 to 4, wherein the isolation is performed by centrifugation or the use of a magnet.

6. The method of any one of claims 1 to 5, wherein the reaction vessel is selected from the group consisting of: a tube, a slide, a plate, a microtitre plate, an array, and a microarray.

7. A method for sample preparation of carrier matrices containing a bacterium of interest for nucleic acid amplification comprising:
(a) contacting antibody-coated particles with a carrier matrix to capture the bacterium of interest, to create a cell/particle complex;
(b) isolating the cell/particle complex by physical means; and
(c) placing the cell/particle complex into a sealed reaction vessel for directly amplifying the nucleic acid wherein the bacterium of interest is lysed under thermocycling conditions, such that lysis is achieved within one or more cycles; wherein the nucleic acid amplification is performed by polymerase chain reaction (PCR) using forward and reverse primers specific for a gene specific for the bacterium of interest wherein the amplified nucleic acid is genomic DNA, wherein said bacterium of interest in the presence of the cell/particle complex of step (b), and determining the presence of amplified nucleic acids and thereby detecting the presence of the bacterium within the sample, wherein step (c) directly succeeds step (b).

8. The method of claim 7, where in the carrier matrix is a food product.

9. The method of claim 8, wherein the food product is ground beef.

10. A method of detecting the presence of a bacterium within a sample, said method comprising:
(a) diluting a sample in a liquid medium that supports growth of the bacterium;
(b) incubating the sample in the liquid medium under conditions and for a time sufficient to allow growth of the bacterium;
(c) contacting the sample in the liquid medium following step (b), or an aliquot thereof, with a carrier having attached antibodies that bind to the bacterium or component thereof for a time sufficient to allow the antibodies to bind the bacterium;
(d) isolating complexes comprising the carrier and the bacterium;
(e) transferring the complex of step (d) to a reaction vessel and directly performing nucleic acid amplification using forward and reverse primers specific for a gene specific for the bacterium being detected wherein the amplified nucleic acid is genomic DNA, wherein said bacterium is bound to the carrier, and wherein the bacterium is lysed under thermocycling conditions, such that lysis is achieved within one or more cycles; and wherein said amplification reaction is performed by PCR, wherein step (e) directly succeeds step (d); and
(f) determining the presence of amplified nucleic acids and thereby detecting the presence of the bacterium within the sample.

11. The method of claim 10, wherein the liquid medium comprises EHEC medium, wherein the antibodies are antibodies specific for *E*. *coli* O157, and wherein the bacterium is *E. coli* O157.

12. The method of claim 10 or 11, wherein the antibodies are associated with magnetic beads and isolating of complexes according to step (d) is performed using a magnet.

13. The method of any one of claims 10 to 12, wherein two or more aliquots of sample in liquid media are each incubated with a different antibody.

14. The method of claim 13, wherein the different antibodies are specific for different bacteria.

15. The method of any one of claims 10 to 14, wherein nucleic acid amplification is performed using two or more sets of forward and reverse primers capable of amplifying a polynucleotide of a bacterium.

16. The method of claim 15, wherein each set of primers is capable of amplifying polynucleotides of different bacteria.

17. The method of claim 15, wherein each set of primers is capable of amplifying polynucleotides of the same bacterium.

18. A method of detecting the presence of a bacterium within a sample, comprising:
(a) in a reaction vessel, contacting a sample with a carrier having attached antibodies that bind to a bacterium or component thereof for a time sufficient to allow the antibody to bind the bacterium;
(b) isolating complexes comprising the antibody and the bacterium in said reaction vessel;
(c) directly performing nucleic acid amplification using: forward and reverse primers specific for a gene specific for the bacterium being detected, wherein said amplified nucleic acid is genomic DNA, wherein said bacterium is bound to the carrier, and wherein the bacterium is lysed under thermocycling conditions, such that lysis is achieved within one or more cycles, and wherein said amplification is performed by polymerase chain reaction (PCR), wherein step (c) directly succeeds step (b); and
(d) determining the presence of amplified nucleic acids and thereby detecting the presence of the bacterium within the sample.

19. The method of any one of claims 1, 7, 10, or 18 wherein the PCR is real-time polymerase chain reaction (real-time PCR).

## Patentansprüche

1. Ein Verfahren zum Nachweis des Vorhandenseins eines Mikroorganismus in einer Probe, wobei der Mikroorganismus ein Bakterium ist, wobei das Verfahren umfasst:
(a) In Kontakt bringen einer Probe mit einem Träger, an den Antikörper gebunden sind, die an das Bakterium oder einen Bestandteil davon binden, für einen Zeitraum, der ausreichend ist, um den Antikörpern zu erlauben, das Bakterium zu binden;
(b) Isolieren des Trägers und jedem damit assoziierten Bakterium, das an die daran gebundenen Antikörper gebunden ist;
(c) Übertragen des Komplexes aus Schritt (b) in ein Reaktionsgefäß und direktes Durchführen einer Nukleinsäureamplifikation unter Verwendung von Vorwärts- und Rückwärts-Primern, die für ein Gen, das für das Bakterium, das nachgewiesen wird, spezifisch ist, spezifisch sind, wobei die amplifizierte Nukleinsäure genomische DNA ist, wobei das Bakterium an den Träger gebunden ist und wobei das Bakterium unter thermozyklischen Bedingungen lysiert wird, so dass die Lyse innerhalb von einem oder mehreren Zyklen erreicht wird; und wobei die Amplifikation durch eine Polymerase-Kettenreaktion (PCR) durchgeführt wird, wobei Schritt (c) Schritt (b) direkt folgt; und
(d) Bestimmen des Vorhandenseins von amplifizierten Nukleinsäuren und **dadurch** Nachweis des Vorhandenseins des Bakteriums in der Probe.

2. Das Verfahren nach Anspruch 1, wobei der Träger ausgewählt wird aus der Gruppe bestehend aus: Kügelchen, Partikeln, Mikropartikeln, unlöslichen Mikropartikeln, magnetischen Kügelchen, unlöslichen Kügelchen, Latexkügelchen, Kunststoffkügelchen, Agarosehydrazid Kügelchen, Agarose Kügelchen, Sepharose und Sephadex.

3. Das Verfahren nach Anspruch 1 oder 2, wobei die Probe ausgewählt wird aus der Gruppe bestehend aus: Nahrungsmittelprodukten, Umweltproben, Wasser und Getränken.

4. Das Verfahren nach einem der Ansprüche 1 bis 3, wobei der Antikörper ausgewählt wird aus der Gruppe bestehend aus: monoklonalen Antikörpern, polyklonalen Antikörpern und Antikörperfragmenten.

5. Das Verfahren nach einem der Ansprüche 1 bis 4, wobei die Isolierung durch Zentrifugation oder die Verwendung eines Magneten durchgeführt wird.

6. Das Verfahren nach einem der Ansprüche 1 bis 5, wobei das Reaktionsgefäß ausgewählt wird aus der Gruppe bestehend aus: einem Röhrchen, einem Objektträger, einer Platte, einer Mikrotiterplatte, einem Array und einem Mikroarray.

7. Ein Verfahren zur Probenvorbereitung von Trägermatrizen, die ein Bakterium von Interesse enthalten, für die Nukleinsäureamplifikation, umfassend:
(a) In Kontakt bringen von Antikörper-beschichteten Partikeln mit einer Trägermatrix, um das Bakterium von Interesse einzufangen, um einen Zell-/Partikel-Komplex zu erzeugen;
(b) Isolieren des Zell-/Partikel-Komplexes durch physikalische Mittel; und
(c) Platzieren des Zell-/Partikel-Komplexes in einem versiegelten Reaktionsgefäß für die direkte Amplifizierung der Nukleinsäure, wobei das Bakterium von Interesse unter thermozyklischen Bedingungen lysiert wird, so dass die Lyse innerhalb von einem oder mehreren Zyklen erreicht wird; wobei die Nukleinsäureamplifikation mittels Polymerase-Kettenreaktion (PCR) unter Verwendung von Vorwärts- und Rückwärts-Primern, die für ein Gen, das für das Bakterium von Interesse spezifisch ist, spezifisch sind, durchgeführt wird, wobei die amplifizierte Nukleinsäure genomische DNA ist, wobei das Bakterium von Interesse in der Gegenwart des Zell-/Partikel-Komplexes aus Schritt (b) ist; und Bestimmen des Vorhandenseins von amplifizierten Nukleinsäuren und **dadurch** Nachweis des Vorhandenseins des Bakteriums in der Probe, wobei Schritt (c) Schritt (b) direkt folgt.

8. Das Verfahren nach Anspruch 7, wobei die Trägermatrix ein Nahrungsmittelprodukt ist.

9. Das Verfahren nach Anspruch 8, wobei das Nahrungsmittelprodukt Rinderhackfleisch ist.

10. Ein Verfahren zum Nachweis des Vorhandenseins eines Bakteriums in einer Probe, wobei das Verfahren umfasst:
(a) Verdünnen einer Probe in einem flüssigen Medium, welches das Wachstum des Bakteriums fördert;
(b) Inkubieren der Probe in dem flüssigen Medium unter Bedingungen und für einen Zeitraum, der ausreichend ist, um das Wachstum des Bakteriums zu erlauben;
(c) In Kontakt bringen der Probe in dem flüssigen Medium oder einem Aliquot davon nach Schritt (b) mit einem Träger, an den Antikörper gebunden sind, die an das Bakterium oder einen Bestandteil davon binden, für einen Zeitraum, der ausreichend ist, um den Antikörpern zu erlauben, das Bakterium zu binden;
(d) Isolieren von Komplexen, die den Träger und das Bakterium umfassen;
(e) Übertragen des Komplexes aus Schritt (d) in ein Reaktionsgefäß und direktes Durchführen einer Nukleinsäureamplifikation unter Verwendung von Vorwärts- und Rückwärts-Primern, die für ein Gen spezifisch sind, das für das Bakterium, das nachgewiesen wird, spezifisch ist, wobei die amplifizierte Nukleinsäure genomische DNA ist, wobei das Bakterium an den Träger gebunden ist, und wobei das Bakterium unter thermozyklisierenden Bedingungen lysiert wird, so dass die Lyse innerhalb von einem oder mehreren Zyklen erreicht wird; und wobei die Amplifikationsreaktion mittels PCR durchgeführt wird, wobei Schritt (e) Schritt (d) direkt folgt; und
(f) Bestimmen des Vorhandenseins von amplifizierten Nukleinsäuren und **dadurch** Nachweis des Vorhandenseins des Bakteriums in der Probe.

11. Das Verfahren nach Anspruch 10, wobei das flüssige Medium EHEC-Medium umfasst, wobei die Antikörper spezifisch für *E. coli* O157 sind, und wobei das Bakterium *E. coli* 0157 ist.

12. Das Verfahren nach Anspruch 10 oder 11, wobei die Antikörper mit magnetischen Kügelchen verbunden sind und die Isolation der Komplexe gemäß Schritt (d) unter Verwendung eines Magneten durchgeführt wird.

13. Das Verfahren nach einem der Ansprüche 10 bis 12, wobei zwei oder mehr Aliquots der Probe in flüssigem Medium jeweils mit einem unterschiedlichen Antikörper inkubiert werden.

14. Das Verfahren nach Anspruch 13, wobei die unterschiedlichen Antikörper für unterschiedliche Bakterien spezifisch sind.

15. Das Verfahren nach einem der Ansprüche 10 bis 14, wobei die Nukleinsäureamplifikation unter Verwendung von zwei oder mehr Sätzen von Vorwärts- und Rückwärts-Primern, die in der Lage sind, ein Polynukleotid eines Bakteriums zu amplifizieren, durchgeführt wird.

16. Das Verfahren nach Anspruch 15, wobei jeder Satz von Primern in der Lage ist, Polynukleotide von verschiedenen Bakterien zu amplifizieren.

17. Das Verfahren nach Anspruch 15, wobei jeder Satz von Primern in der Lage ist, Polynukleotide desselben Bakteriums zu amplifizieren.

18. Ein Verfahren zum Nachweis des Vorhandenseins eines Bakteriums in einer Probe, umfassend:
(a) in Kontakt Bringen einer Probe mit einem Träger, an den Antikörper gebunden sind, die an ein Bakterium oder einen Bestandteil davon binden, in einem Reaktionsgefäß für einen Zeitraum, der ausreichend ist, um dem Antikörper zu erlauben, das Bakterium zu binden;
(b) Isolieren von Komplexen, die den Antikörper und das Bakterium umfassen, in dem Reaktionsgefäß:
(c) direktes Durchführen einer Nukleinsäureamplifikation unter Verwendung von Vorwärts- und Rückwärts-Primern, die für ein Gen spezifisch sind, das für das Bakterium, das nachgewiesen wird, spezifisch ist, wobei die amplifizierte Nukleinsäure genomische DNA ist, wobei das Bakterium an den Träger gebunden ist und wobei das Bakterium unter thermozyklischen Bedingungen lysiert wird, so dass die Lyse innerhalb von einem oder mehreren Zyklen erreicht wird; und wobei die Amplifikation mittels Polymerase-Kettenreaktion (PCR) durchgeführt wird, wobei Schritt (c) Schritt (b) direkt folgt; und
(d) Bestimmen des Vorhandenseins von amplifizierter Nukleinsäure und **dadurch** Bestimmung des Vorhandenseins des Bakteriums in der Probe.

19. Das Verfahren nach einem der Ansprüche 1, 7, 10 oder 18, wobei die PCR Echtzeit Polymerase-Kettenreaktion (Echtzeit PCR) ist.

## Revendications

1. Procédé pour détecter la présence d'un microorganisme à l'intérieur d'un échantillon, dans lequel le microorganisme est une bactérie, le procédé comprenant les étapes suivantes :
(a) mettre en contact un échantillon avec un porteur comportant des anticorps fixés qui se lient à la bactérie ou à un élément de celle-ci pendant une durée suffisante pour permettre aux anticorps de se lier à la bactérie ;
(b) isoler le porteur et toute bactérie associée liée aux anticorps fixés ;
(c) transférer le complexe de l'étape (b) vers un récipient à réaction et réaliser directement une amplification d'acide nucléique en utilisant des amorces avant et inverse spécifiques à un gène spécifique à la bactérie détectée, dans lequel l'acide nucléique amplifié est un ADN génomique, dans lequel ladite bactérie est liée au porteur, et dans lequel la bactérie est lysée dans des conditions de cycle thermique, de sorte qu'une lyse soit réalisée en un ou plusieurs cycles ; et dans lequel ladite amplification est réalisée par réaction en chaîne par polymérase (PCR - Polymerase Chain Reaction), dans lequel l'étape (c) succède directement à l'étape (b) ; et
(d) déterminer la présence d'acides nucléiques amplifiés puis détecter ainsi la présence de la bactérie à l'intérieur de l'échantillon.

2. Procédé selon la revendication 1, dans lequel le porteur est sélectionné dans le groupe constitué de billes, de particules, de microparticules, de microparticules insolubles, de billes magnétiques, de billes insolubles, de billes en latex, de billes en plastique, de billes d'hydrazide-agarose, de billes d'agarose, de Sépharose et de Sephadex.

3. Procédé selon la revendication 1 ou 2, dans lequel l'échantillon est sélectionné dans le groupe constitué de produits alimentaires, d'échantillons environnementaux, d'eau et de boissons.

4. Procédé selon l'une quelconque des revendications 1 à 3, dans lequel l'anticorps est sélectionné dans le groupe constitué d'anticorps monoclonaux, d'anticorps polyclonaux et de fragments d'anticorps.

5. Procédé selon l'une quelconque des revendications 1 à 4, dans lequel l'isolation est réalisée par centrifugation ou par l'utilisation d'un aimant.

6. Procédé selon l'une quelconque des revendications 1 à 5, dans lequel le récipient à réaction est sélectionné dans le groupe constitué d'un tube, d'une lame, d'une plaque, d'une plaque de microtitration, d'un réseau et d'un microréseau.

7. Procédé pour la préparation d'un échantillon de matrices de support contenant une bactérie d'intérêt pour l'amplification d'acide nucléique comprenant les étapes suivantes :
(a) mettre en contact des particules recouvertes d'anticorps avec une matrice de porteur pour capturer la bactérie d'intérêt, afin de créer un complexe cellule/particule ;
(b) isoler le complexe cellule/particule par des moyens physiques ; et
(c) placer le complexe cellule/particule dans un récipient à réaction fermé hermétiquement pour amplifier directement l'acide nucléique, dans lequel la bactérie d'intérêt est lysée dans des conditions de cycle thermique, de sorte qu'une lyse soit réalisée en un ou plusieurs cycles ; dans lequel l'amplification d'acide nucléique est réalisée par réaction en chaîne par polymérase (PCR) en utilisant des amorces avant et inverse spécifiques à un gène spécifique à la bactérie d'intérêt, dans lequel l'acide nucléique amplifié est un ADN génomique, dans lequel ladite bactérie d'intérêt est en présence du complexe cellule/particule de l'étape (b), et déterminer la présence d'acides nucléiques amplifiés puis détecter ainsi la présence de la bactérie à l'intérieur de l'échantillon, dans lequel l'étape (c) succède directement à l'étape (b).

8. Procédé selon la revendication 7, dans lequel la matrice de porteur est un produit alimentaire.

9. Procédé selon la revendication 8, dans lequel le produit alimentaire est du boeuf haché.

10. Procédé pour détecter la présence d'une bactérie à l'intérieur d'un échantillon, ledit procédé comprenant les étapes suivantes :
(a) diluer un échantillon dans un milieu liquide qui favorise le développement de la bactérie ;
(b) faire incuber l'échantillon dans le milieu liquide dans des conditions et pendant une durée qui permettent le développement de la bactérie ;
(c) mettre en contact l'échantillon dans le milieu liquide après l'étape (b), ou une aliquote de celui-ci, avec un porteur comportant des anticorps fixés qui se lient à la bactérie ou à un élément de celle-ci pendant une durée suffisante pour permettre aux anticorps de se lier à la bactérie ;
(d) isoler des complexes comprenant le porteur et la bactérie ;
(e) transférer le complexe de l'étape (d) vers un récipient à réaction et réaliser directement une amplification d'acide nucléique en utilisant des amorces avant et inverse spécifiques à un gène spécifique à la bactérie détectée, dans lequel l'acide nucléique amplifié est un ADN génomique, dans lequel ladite bactérie est liée au porteur, et dans lequel la bactérie est lysée dans des conditions de cycle thermique, de sorte qu'une lyse soit réalisée en un ou plusieurs cycles ; et dans lequel ladite réaction d'amplification est réalisée par PCR, dans lequel l'étape (e) succède directement à l'étape (d) ; et
(f) déterminer la présence d'acides nucléiques amplifiés et détecter ainsi la présence de la bactérie à l'intérieur de l'échantillon.

11. Procédé selon la revendication 10, dans lequel le milieu liquide comprend un milieu d'Escherichia coli entérohémorragique, dans lequel les anticorps sont des anticorps spécifiques à l'Escherichia coli 0157, et dans lequel la bactérie est l'Escherichia coli 0157.

12. Procédé selon la revendication 10 ou 11, dans lequel les anticorps sont associés à des billes magnétiques et l'isolation de complexes conformément à l'étape (d) est réalisée en utilisant un aimant.

13. Procédé selon l'une quelconque des revendications 10 à 12, dans lequel au moins deux aliquotes d'échantillon dans des milieux liquides sont chacune incubée avec un anticorps différent.

14. Procédé selon la revendication 13, dans lequel les anticorps différents sont spécifiques à des bactéries différentes.

15. Procédé selon l'une quelconque des revendications 10 à 14, dans lequel l'amplification d'acide nucléique est réalisée en utilisant au moins deux ensembles d'amorces avant et inverse pouvant amplifier un polynucléotide d'une bactérie.

16. Procédé selon la revendication 15, dans lequel chaque ensemble d'amorces peut amplifier des polynucléotides de bactéries différentes.

17. Procédé selon la revendication 15, dans lequel chaque ensemble d'amorces peut amplifier des polynucléotides de la même bactérie.

18. Procédé pour détecter la présence d'une bactérie à l'intérieur d'un échantillon, comprenant les étapes suivantes :
(a) dans un récipient à réaction, mettre en contact un échantillon avec un porteur comportant des anticorps fixés qui se lient à une bactérie ou à un élément de celle-ci pendant une durée suffisante pour permettre à l'anticorps de se lier à la bactérie ;
(b) isoler des complexes comprenant l'anticorps et la bactérie dans ledit récipient à réaction ;
(c) réaliser directement une amplification d'acide nucléique en utilisant : des amorces avant et inverse spécifiques à un gène spécifique à la bactérie détectée, dans lequel ledit acide nucléique amplifié est un ADN génomique, dans lequel ladite bactérie est liée au porteur, et dans lequel la bactérie est lysée dans des conditions de cycle thermique, de sorte qu'une lyse soit réalisée en un ou plusieurs cycles, et dans lequel ladite amplification est réalisée par réaction en chaîne par polymérase (PCR - Polymerase Chain Reaction), dans lequel l'étape (c) succède directement à l'étape (b) ; et
(d) déterminer la présence d'acides nucléiques amplifiés puis détecter ainsi la présence de la bactérie à l'intérieur de l'échantillon.

19. Procédé selon l'une quelconque des revendications 1, 7, 10 ou 18 dans lequel la PCR est une réaction en chaîne par polymérase en temps réel (PCR en temps réel).
